# EUROPEAN PATENT APPLICATION

(11) **EP 4 699 573 A2**
(43) Date of publication of application: **25.02.2026**
(21) Application number: 25205813.6
(22) Date of filing: 22.07.2021
(51) Int. Cl.: A61F 2/24

(54) **DOCKING STATION FOR HEART VALVE PROSTHESIS**

(30) Priority: 23.07.2020 US 202063055809 P
(62) Divisional of application: 21755241.3
(71) Applicant: Edwards Lifesciences Corporation, Irvine, CA 92614 (US)
(72) Inventor: Tran, Tri D., IRVINE CA, 92614 (US); Saiduddin, Adeeb, IRVINE CA, 92614 (US); Cayabyab, Ronaldo C., IRVINE CA, 92614 (US)
(74) Representative: Eisenführ Speiser

(57) **Abstract**

An anchoring device that can be positioned within a native valve, such as the native mitral valve, to secure a replacement prosthetic valve in place. The anchoring device can comprise a docking Station formed of an elastic tube-like member defining a generally coiled shape. The docking Station can have an atrial or stabilization turn, a leading or encircling turn, and a central region. In many embodiments, the docking device possess a plurality of cuts on opposing sides of the tube-like member to allow biasing of the member.

## Description

### CROSS-REFERENCE TO RELATED APPLICATIONS

The current application claims priority to U.S. Provisional Patent Application No. 63/055,809, filed July 23, 2020; the disclosure of which is hereby incorporated by reference in its entirety.

### FIELD OF THE INVENTION

The present invention relates to anchoring valvular prosthetics. More specifically, the present invention relates to a reduced-turn docking device to anchor a valvular prosthetic.

### BACKGROUND

Heart valve regurgitation can occur when the heart leaflets do not completely close when the heart contracts. For example, mitral valve regurgitation occurs when blood flows from the left ventricle back through the mitral valve and into the left atrium when the ventricle contracts.

In some instances, regurgitation occurs due to disease of the valve leaflets (e.g., primary, or "organic" regurgitation). Regurgitation can also be cause by dilatation of the left ventricle, which can lead to secondary dilatation of the mitral valve annulus. Dilation of the annulus spreads the mitral valve leaflets apart and creates poor tip cooptation and secondary leakage, or so-called "functional regurgitation."

Primary regurgitation can be corrected by attempting to remodel the native leaflets, such as with clips, sutures, hooks, etc., to allow them to close completely when the heart contracts. When the disease is too far advanced, the entire valve may need to be replaced with a prosthesis, e.g., mechanical or biologic. Based on the success of catheter-based aortic valve replacement, it would be beneficial to have options usable to replace the mitral valve non- invasively using similar types of replacement valves.

Unlike the aortic valve, however, the mitral valve annulus does not provide a good landmark for positioning a replacement mitral valve. The bulk of the aortic annulus is generally increased in the presence of degenerative disease associated with calcium formation, thus making it easier to properly secure a replacement aortic valve in place due to the reduced cross- sectional area of the aortic annulus. However, within mitral valves experiencing regurgitation, the mitral valve annulus is generally thinner not thicker, wider not narrower. The thinner, wider mitral valve annulus makes it relatively more difficult to properly seat a replacement mitral valve in the native mitral valve annulus. Further, the aortic annulus is anatomically pronounced, providing a larger "bump" to which a replacement aortic valve can be secured, whereas the smoother transition from the left atrium to the left ventricle defined by the mitral valve annulus makes it more difficult to properly anchor a replacement mitral valve in place.

Further, the larger mitral valve annulus makes it difficult to securely implant current percutaneously delivered valves in the native mitral position. Current replacement valves, typically designed to replace aortic valves, are limited in the amount of radial expansion they can undergo during deployment and implantation. Due to differences in the native aortic valve and native mitral valve anatomies, the collapsed delivery profile of a replacement aortic valve would require an increased profile to deploy a replacement aortic valve in a mitral valve annulus. However, that would make endovascular delivery more dangerous for the patient and more difficult to navigate the vasculature with a larger diameter delivery system. Further, self expanding stents that cause the valve to become anchored to the valve annulus may not be feasible for repair of a mitral valve due to the possibility that the self-expanding stent may occlude the left ventricle outflow tract for the adjacent aortic valve.

Despite certain advances in mitral valve replacement, there remains a need for new effective and safe anchoring devices that can be positioned near or within the native mitral valve and that is adapted to secure a replacement mitral valve in place. Past attempts for anchoring devices include systems that provide structures lying above the mitral plane to prevent an anchoring device from slipping or otherwise migrating downwards into a ventricle. Additional attempts utilize coils possessing multiple turns to encircle native valve anatomy (e.g., leaflets, chordae, etc.) and are held in place by friction and/or radially inward pressure on the native anatomy.

### SUMMARY OF THE INVENTION

This summary is meant to provide some examples and is not intended to be limiting of the scope of the invention in any way. For example, any feature included in an example of this summary is not required by the claims, unless the claims explicitly recite the features. Various features and steps as described elsewhere in this disclosure may be included in the examples summarized here, and the features and steps described here and elsewhere can be combined in a variety of ways.

In one embodiment, a docking device for docking a prosthetic valve at a native heart valve includes a coil constructed of a hypotube and extending along a central axis, including a leading turn, a central region, and a stabilization turn, where the central region possesses approximately one turn, the leading turn extends from one end of the central region and has a diameter greater than a diameter of the central region, and the stabilization turn has a diameter greater than the diameter of the central region and extends from an opposing end of the central region from the leading turn.

In a further embodiment, the hypotube includes a plurality of cuts to allow biasing of the hypotube.

In another embodiment, at least one of a width and a depth of cuts on one side of the hypotube allow for biasing of the hypotube.

In a still further embodiment, at least one of a width and a depth of cuts on one side of the hypotube are varied along the length of the hypotube allowing for varied biasing of the hypotube along its length.

In still another embodiment, a pitch is varied between cuts to allow for biasing of the hypotube.

In a yet further embodiment, the pitch varies between 1.5mm and 2.33mm.

In yet another embodiment, the stabilization turn has a pitch of approximately 2.33mm.

In a further embodiment again, the central region has a pitch of approximately 1.5mm.

In another embodiment again, the central region has a pitch of approximately 2.0mm.

In a further additional embodiment, the encircling turn has a pitch of between approximately 1.5mm and approximately 1.92mm.

In another additional embodiment, the encircling turn includes a proximal subsection and a distal subsection, where the proximal subsection has a pitch of approximately 1.5mm, and the distal subsection has a pitch of approximately 1.92mm.

In a still yet further embodiment, the coil further includes a transition zone between the central region and the stabilization turn.

In still yet another embodiment, at least one of a pitch and a depth of cuts are varied along the length of the transition zone.

In a still further embodiment again, the coil further includes a transition zone between the central region and the encircling turn.

In still another embodiment again, at least one of a pitch and a depth of cuts are varied along the length of the transition zone.

In a still further additional embodiment, the docking device further includes an atraumatic covering disposed on the coil.

In still another additional embodiment, the atraumatic covering is constructed of PET or ePTFE.

In a yet further embodiment again, the coil is constructed of nitinol.

In yet another embodiment again, the docking device further includes an actuating wire connected to a distal tip of the leading turn and running inside the hypotube.

In a yet further additional embodiment, the docking device further includes an interlocking disconnect device connected to a proximal end of the docking device.

In yet another additional embodiment, the docking device further includes a pawl disposed on an internal surface of the hypotube and a tooth disposed on the actuating wire, where movement of the actuating wire beyond a certain point causes the pawl and tooth to engage.

In a further additional embodiment again, a system to deliver a docking device at a native valve includes a delivery catheter, a docking device as described herein, the docking device having an end portion at the end of the stabilization turn located opposite the central region, a pusher shaft disposed in the delivery catheter and coupled to the end portion of the docking device, and an actuating wire connected to a distal end of the docking device and running coaxially with the pusher shaft, where the system is configured such that the pusher shaft and the actuating wire are configured to operate in parallel.

In another additional embodiment again, the system further includes an actuating assembly, where the pusher shaft and the actuating wire are controlled via a control assembly.

In a still yet further embodiment again, the actuating assembly includes a generally Y-shaped connector.

In still yet another embodiment again, the generally Y-shaped connector includes a straight section and a branch, where the actuating wire extends to the end of the straight section.

In a still yet further additional embodiment, the system further includes an actuating handle connected to the actuating wire, where manipulation of the actuating handle manipulates a bias of the docking device.

In still yet another additional embodiment, the system further includes an interlocking disconnect device disposed between the pusher shaft and the docking device.

In a yet further additional embodiment again, the interlocking disconnect device includes a pusher component and a dock component, each including a connecting portion and an interlocking portion, where the interlocking portion of the pusher component and the interlocking portion of the wire component interleave.

In yet another additional embodiment again, the interlocking disconnect device forms a central pore, where the actuating wire passes through the central pore.

In a still yet further additional embodiment again, the system further includes a locking mechanism to lock the actuating wire in place.

In still yet another additional embodiment again, the dock component is affixed to the docking device and the pusher component is affixed to the pusher shaft.

In another further embodiment, the system further includes a linear rack including a tooth affixed to the actuating wire and a pawl affixed to an inner surface of the docking device, where the linear rack and pawl are configured to interact when the actuating wire is manipulated beyond a certain point.

In still another further embodiment, a method to deliver a docking device includes advancing a delivery catheter to a native valve and advancing a docking device disposed within the delivery catheter through a valve commissure and around valvular leaflets.

In yet another further embodiment, the method further includes actuating an actuating wire disposed within the docking device to maneuver the docking device around the valvular leaflets.

In another further embodiment again, the method further includes actuating an actuating wire to achieve a desired geometry of the docking device.

Another further additional embodiment, the method further includes actuating an actuating wire to a point that locks the actuating wire in place.

In yet another further additional embodiment again, the method further includes severing the actuating wire.

In various embodiments, the methods can be performed on a living animal or on a non-living cadaver, cadaver heart, simulator (e.g. with the body parts, tissue, etc. being simulated), anthropomorphic ghost, etc.

The foregoing and other objects, features, and advantages of the disclosed technology will become more apparent from the following detailed description, which proceeds with reference to the accompanying figures.

### BRIEF DESCRIPTION OF THE DRAWINGS

The description and claims will be more fully understood with reference to the following figures and data graphs, which are presented as exemplary embodiments of the invention and should not be construed as a complete recitation of the scope of the invention.
Figure 1 illustrates a perspective view of an example embodiment of docking device or core of a docking device with a reduced number of functional turns in accordance with an embodiment of the invention.
Figure 2A-2D illustrate example segments of a docking device capable of being manipulated and/or maneuvered in accordance with an embodiment of the invention.
Figures 2E-2G illustrate examples of a docking device with cut segments to allow the docking device to be manipulated and/or maneuvered in accordance with an embodiment of the invention.
Figure 3 illustrates example embodiments of a handle assembly for a delivery system in accordance with various embodiments.
Figures 4A-4B illustrate perspective views of components of an example interlocking disconnect device in accordance with various embodiments.
Figure 4C illustrates a perspective view of an example interlocking disconnect device in accordance with various embodiments.
Figures 5A-5B illustrate a ratcheting system to lock an actuating wire in accordance with various embodiments.
Figure 6A illustrates a pusher shaft and docking device interface in accordance with various embodiments.
Figures 6B-6D illustrate exemplary cross sections of a detachment section of an actuating wire in accordance with various embodiments.

### DETAILED DESCRIPTION

Disclosed herein are various embodiments of systems, apparatuses, methods, etc., including anchoring or docking devices, which can be used in conjunction with expandable prosthetic valves (e.g., transcatheter heart valves (THV)) at a native valve annulus (e.g., mitral or tricuspid valve annulus), in order to more securely implant and hold the prosthetic valve at the implant site. Anchoring/docking devices according to many embodiments provide or form a more circular and/or stable anchoring site, landing zone, or implantation zone at the implant site, in which prosthetic valves can be expanded or otherwise implanted. Many of these docking devices and respective prosthetic valves have circular valve frames or stents can be expanded or otherwise implanted into locations with naturally circular cross sections. However, further embodiments of docking devices have geometries of natural valves (e.g., oblong, ovular, longitudinally curved, etc.) which are more appropriate for non-circular and/or non-cylindrical anatomies. In addition to providing an anchoring site for the prosthetic valve, the anchoring/docking devices can be sized and shaped to cinch or draw the native valve (e.g., mitral, tricuspid, etc.) anatomy radially inwards. In this manner, one of the main causes of valve regurgitation (e.g., functional mitral regurgitation), specifically enlargement of the heart (e.g., enlargement of the left ventricle, etc.) and/or valve annulus, and consequent stretching out of the native valve (e.g., mitral, etc.) annulus, can be at least partially offset or counteracted. Some embodiments of the anchoring or docking devices further include features which, for example, are shaped and/or modified to better hold a position or shape of the docking device during and/or after expansion of a prosthetic valve therein. By providing such anchoring or docking devices, replacement valves can be more securely implanted and held at various valve annuluses, including at the mitral annulus which does not have a naturally circular cross- section.

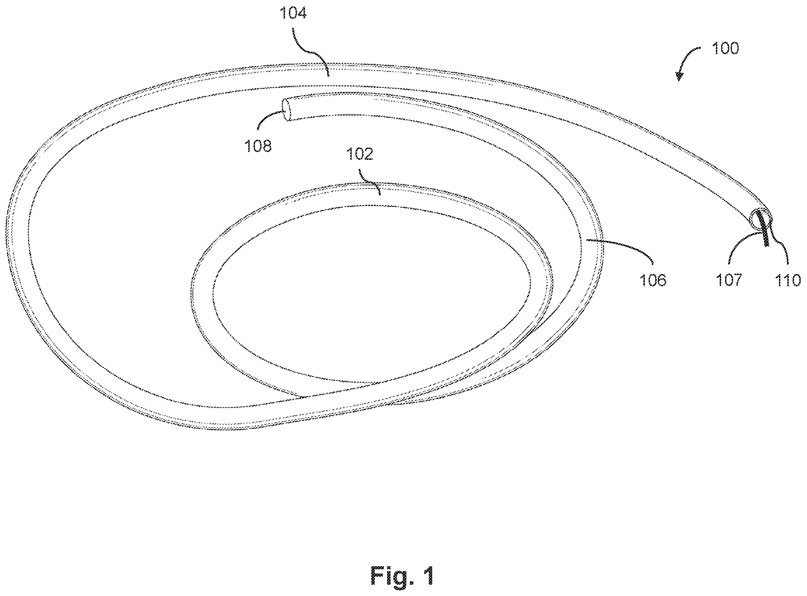

Turning to Figure 1, this figure provides representative embodiments of a docking device 100. Figure 1 is representative of a core of a docking device that can include a covering or other addition onto said core to form a docking device. It is possible that certain docking devices in an atrio-ventricular position may migrate further or deeper into the ventricle post deployment of the docking device than desired. It may be beneficial to avoid too much "dock drop" to help avoid paravalvular leakage, e.g., sealing the docking device and prosthetic valve seal together higher on the native leaflets may help prevent paravalvular leaks that might occur if done lower on the chordae tendineae. Additionally, higher placement (e.g., by avoiding dock drop) may help long term stability of the valve, because the leaflets are thicker and more robust near the annulus rather than near their distal end, thus anchoring at or near the annulus is expected to be stronger and provide longer term stability. Also, higher placement of the prosthetic valve may help avoid abrasion of the valve against the native tissue (e.g., against the native leaflets and/or chordae tendineae), by making the artificial valve less likely to rub against the leaflets and/or chordae tendineae.

Paravalvular leakage can occur due to a number of causes, including where the native annulus is too large in comparison the prosthetic valve; the commissural leaflets are too short and/or are damaged; the implantation of a docking device did not completely capture the native leaflets; the crossing of a docking device from one side of the valve to the other causes a small gap (e.g., in the commissure); the placement of a prosthetic valve is too biased toward the lateral, anterior, posterior, and/or medial sides of a native valve; and/or anatomical abnormalities in certain patients (e.g., clefts, those associated with degenerative mitral regurgitation, etc.). Various embodiments of this disclosure are designed to compensate for, avoid, reduce, and/or obviate many of these issues, including by holding the dock up on both sides of the native mitral valve (thus reducing and/or inhibiting dock drop by maintaining the dock and prosthetic valve close to the native annulus plane), by creating a better seal around a prosthetic valve, by creating a better seal above the native annulus, etc.

In the example illustrated in Figure 1, numerous embodiments of docking device 100 possess a roughly coiled shape extending along a central axis with a central region 102, stabilization coil (also referred to as stabilization turn or atrial turn) 104, and an encircling coil (or turn) 106. The central region 102 can be continuous and can extend generally helically, with various differently sized and shaped sections, as described in greater detail below. The docking device 100 shown in Figure 1 is configured to fit at the mitral position but can be shaped and/or adapted similarly or differently in other embodiments for better accommodation at other native valve positions as well, such as at the tricuspid valve.

Various factors can contribute to the total retention force between the docking device 100 and the prosthetic valve held therein. Factors that contribute to retention force of various embodiments include, for example, an inner diameter of the functional coils, friction force (e.g., between the coils and the prosthetic valve), and the strength of the prosthetic valve and the radial force the valve applies on the coil. In Figure 1, the docking device 100 is designed to have a low turns or coils in central region 102 while maintaining retention forces on native anatomy and/or a prosthetic valve. In some embodiments, the number of turns is approximately 1 turn (±0.125 turns), approximately 1.25 (±0.125 turns), approximately 1.5 (±0.125 turns), approximately 1.75 (±0.125 turns), approximately 2 (±0.125 turns), approximately 2.25 (±0.125 turns), approximately 2.5 (±0.125 turns), approximately 2.75 (±0.125 turns), or approximately 3 (±0.125 turns) or more turns in the central region. In embodiment possessing greater than 1 turn in the central region 102, the turns can be similarly sized and shaped or vary in size and/or shape. The central region 102 of the docking device 100 serves as the main landing region or holding region for holding the expandable prosthetic valve when the docking device 100 and the valve prosthesis are implanted into a heart. While many embodiments possess 1 turn, or approximately 1 turn, certain embodiments will possess greater or fewer turns (or fractions of turns), depending for example, on the patient's anatomy, the amount of vertical contact desired between the docking device 100 and the valve prosthesis (e.g., transcatheter heart valve or THV), and/or other factors. The coiled portion or coil(s) of the central region 102 can also be referred to as the "functional coils" or "functional turns" since the properties of these coils contribute the most to the amount of retention force generated between the valve prosthesis, the docking device 100, and the native mitral leaflets and/or other anatomical structures.

Figure 1 illustrates an embodiment with a flat stabilization coil (or turn) 104 having a diameter greater than the diameter of the central region 102 and extends from at an opposing end of a central region 102 as an encircling (or leading) turn 106. In many embodiments, the stabilization coil 104 sits on a plane of the native valve (e.g., on a native mitral plane or a native tricuspid plane). In such embodiments, the stabilization coil 104 is designed to be larger than the opening of the native valve (e.g., of the mitral valve or tricuspid valve), yet not so large that the stabilization coil 104 does not sit on a plane of the native valve. The stabilization coil 104 can form a continuous curve, such as illustrated in Figure 1 or can be flared out or biased toward the posterior wall of the atrium, thus taking advantage of the posterior shelf, to prevent the docking device 100 from falling into the ventricle prior to deployment of an artificial or prosthetic valve therein. Additionally, in certain flat embodiments, the stabilization coil will have a smooth cover to prevent trauma to native anatomy in regions that exhibit relative motion (e.g., where the docking device 100 crosses from the atrium to the ventricle through the mitral valve).

Additional embodiments possess different geometries for the stabilization turn 102, including a design to create a plane formed by at least three points of contact in the atrium. These three anchoring points or points of contact are the posterior shelf of the native valve (e.g., of the mitral valve, etc.), the anterior wall of the atrium, and either an atrial appendage of the atrium or the lateral shelf of the native valve.

Further embodiments possess stabilization coils 104 hybrid dock design is illustrated that is configured to improve prevention and/or inhibition of paravalvular leakage. In embodiments of this hybrid docking device 100, the stabilization coil 104 is designed to create a closer ring around a deployed prosthetic valve, thus helping to seal the valve and preventing paravalvular leakage. In some embodiments, this sealing effect is maximized by offsetting radially outwardly the dock core from the maximum valve outer diameter plus half the cross section of the docking device. In some embodiments, the stabilization coil 104 allows for the largest outer diameter (e.g., optimal contact with the atrium) to help also with dock drop prior to prosthetic valve deployment. Additionally, in some embodiments, the docking device is configured such that, after prosthetic valve deployment, the stabilization coil will be flush with the outer diameter of the implanted and expanded prosthetic valve. This hybrid design can be manufactured to rely on different prosthetic valve outer diameters to prevent paravalvular leakage close around the prosthetic valve.

Many embodiments possess an encircling turn 106 (or leading turn) extending from a leading end of central region 102 to guide or navigate the turns/coils of central region 102 around and/or through the chordae tendineae geometry, and most importantly, adequately around both native leaflets of the native valve (e.g., the native mitral valve, tricuspid valve, etc.). Once the distal tip 108 of encircling turn 106 is navigated around the desired native anatomy, the remaining coils of the docking device 100 can also be guided around the same features. In some embodiments, the size of the other coils can be reduced sufficiently to cause the corralled anatomical features to be pulled radially inwardly or slightly radially inwardly. Meanwhile, the length of the enlarged encircling turn 106 can be kept relatively short, to prevent or avoid obstruction or interference of the flow of blood along the ventricular outflow tract by the encircling turn 106. For example, in one embodiment, the enlarged encircling turn 106 extends for only about half a loop or rotation.

In many embodiments, the ability of a docking device 100 to possess a reduced number of turns in central region 102 is due to the construction of the core of docking device 100. In various embodiments, the core of a docking device 100 embodiments is constructed of a tube, such as a hypotube, or another elongated and hollow member.

In additional embodiments, an actuating wire 107 runs longitudinally through the tube of docking device 100 from distal tip 108 through proximal opening 110. In many of these embodiments the actuating wire is securely connected to distal tip 108. The actuating wire allows an individual to maneuver a docking device 100 and/or manipulate the geometry of a docking device 100 during installation of docking device 100.

A tube in accordance with various embodiments has an outer diameter to provide overall strength and/or retention force and an inner diameter wide enough to allow for an actuating wire to pass. In certain embodiments the inner diameter allows for an actuating wire 107 to be displaced from a central axis running longitudinally trough docking device 100, as a coaxial actuating wire 107 may be ineffective in manipulating docking device 100. In certain embodiments, the outer diameter of docking device 100 is narrow enough to prevent contact with ventricular walls. The outer diameter of tubes of certain embodiments is approximately 1.2 mm (e.g., ±0.5 mm) to approximately 4 mm (e.g., ±0.5 mm).

Suitable materials for a docking device include a core constructed of a flexible and biocompatible material. In some embodiments, the core is constructed of a shape memory material, including, but not limited to, shape memory alloy, shape memory metal, nitinol, stainless steel, and other materials known in the art. A number of embodiments include a covering disposed on the core to be less traumatic or atraumatic to native anatomy. In some embodiments, the covering is PET, ePTFE, including woven, braided, knitted, and electrospun materials made of PET and/or ePTFE. Additional embodiments use a covering constructed of a biocompatible and/or bioimplantable material. Further embodiments use additional atraumatic materials, including expandable gels, hydrophilic materials and/or coatings, and hydrogels.

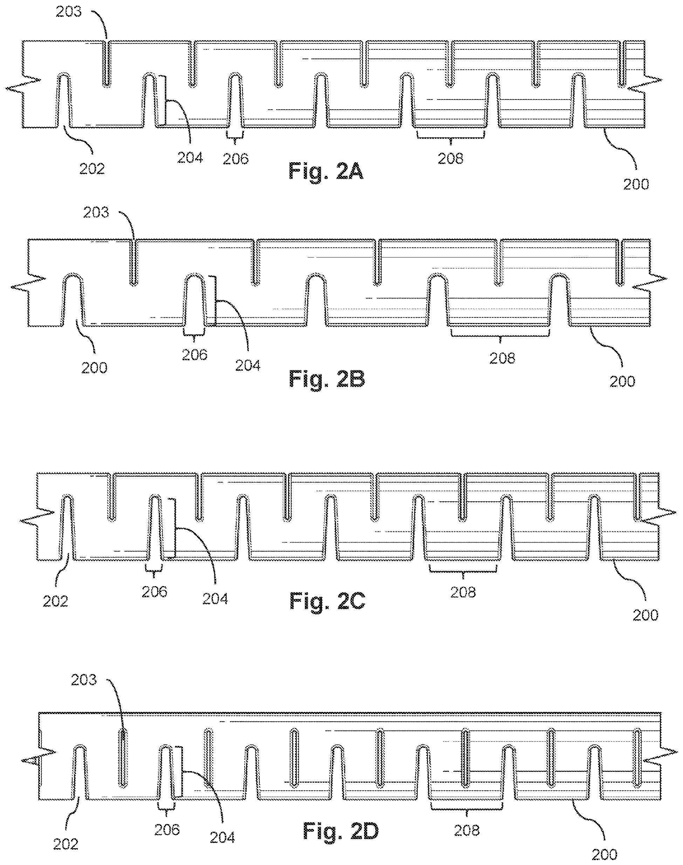

Turning to Figures 2A-2D, segments of a tube 200 of many embodiments are illustrated. In various embodiments, tube 200 forms the basis for a docking device core, such as illustrated in Figure 1. The ability of many embodiments to maneuver via an actuating wire is granted by plurality of primary cuts or grooves 202 cut into one side of tube 200 to allow for deflection of tube 200 toward primary cuts 202. Additional embodiments possess a plurality of secondary cuts or grooves 203 located on one or more additional sides to allow for flexibility of tube 200. For example, as illustrated in Figures 2A-2C, secondary cuts 203 are offset and located approximately 180° in rotation from primary cuts 202. However, Figure 2D illustrates secondary cuts 203 that are offset and located approximately 90° in rotation from primary cuts 202. In various embodiments, a plurality of secondary cuts 203 nearly encircle tube 200, such that each secondary cut 203 extends nearly 180° (e.g., 170°-175°) around tube 200. In some embodiments, the plurality of cuts 202, 203 are made in tube 200 by cutting into tube 200, including by laser cutting specific cuts 202, 203 into tube 200.

While cuts 202, 203 are illustrated as generally triangular in Figures 2A-2D, some embodiments of cuts 202, 203 include additional shapes, such as a bulbous or rounded apex. A bulbous or rounded apex can prevent mechanical stress at the apex of a cut that could lead to cracking, splitting, or other mechanical failure in a tube or docking device. Additionally, a bulbous or rounded apex can allow for additional movement or flexion in tube 200 by granting additional freedom of movement in cuts 202, 203.

As seen in Figures 2A-2C, altering width and/or depth of cuts on one side of tube 200 can allow for biasing of tube 200 to one side upon the introduction of a longitudinal force, such as through increasing tension on an actuating wire (e.g., Figure 1, actuating wire 107) running through tube 200. Additionally, the inclusion of cuts and an actuating wire allows for the creation of a deflectable tip or "active tip" - e.g., a maneuverable tip that allows a user to navigate a docking device through native anatomy (e.g., leaflets, chordae, etc.) to aid in encircling the tissue sufficiently to secure a docking device. Additionally, various embodiments possess primary cuts 202 having differences in depth 204, width 206, and/or spacing distance (also referred to as pitch) 208 to allow for various geometries upon cinching a docking device via increased tension on an articulating wire.

In many embodiments, a single length of tube 200 has varying depths 204, widths 206, and/or spacing distances 208 of cuts along the length of tube 200, such that under tension, the tube can form the shape of a docking device (e.g., Figure 1, docking device 100). For example, in reference to Figure 1, central region 102 may have wider cuts 202 thus allowing for increased bias, thus a narrower diameter of the central region, as compared to encircling turn 106 and/or stabilization coil 104. In further embodiments, depths and/or widths of cuts 202, 203 vary along the length of tube 200, such as to transition between various sections or segments of a docking device. For example, cuts 202, 203 vary to allow a transition between a stabilization coil 104 and a central region 102 and/or between an encircling coil 106 and a central region 102.

As noted herein, cuts 202, 203 can provide a level of flexibility to a docking device during implantation. However, a longitudinal force applied via an actuating wire can provide sufficient force to lock a docking device into a specific conformation. By locking a docking device into a specific conformation, the docking device can be retained at the implantation site (e.g., at a valvular annulus and/or surrounding native valvular leaflets).

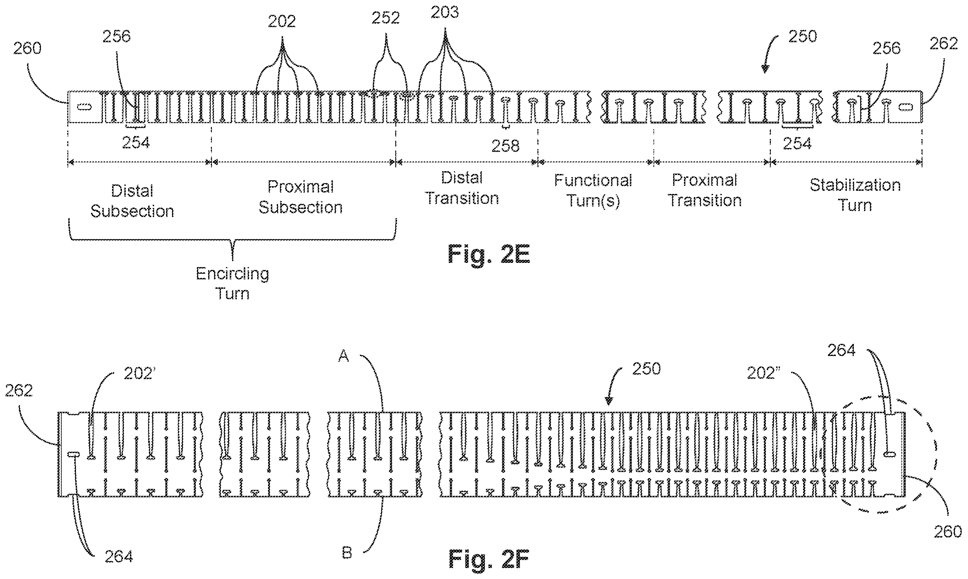

Figures 2E-2G illustrate a longitudinal view of a docking device 250 incorporating primary cuts 202 and secondary cuts 203 in accordance with various embodiments how various cuts can be implemented into a tubular body of a docking device 250 to achieve the overall shape or structure of the docking device. Figure 2E illustrates a longitudinal view of a docking device 250. Docking device 250 includes a plurality of primary cuts 202 along one side to allow for biasing or bending of docking device 250 in the direction of the primary cuts 202. Additionally, secondary cuts 203 are illustrated at a position between each of primary cut 202 and offset by 90°. As shown in Figure 2E, both primary cuts 202 and secondary cuts 203 possess bulbous or rounded apices 252. As noted herein, bulbous or rounded apices 252 prevent stresses from accumulating at the apex of a cut that may lead to cracking. Additionally, such bulbous or rounded apices 252 allow for additional flexion in a docking device 250 granted by additional space provided by such bulbous or rounded apices 252.

As noted above, docking device 250 can be defined by various segments or sections depending along its length, including a stabilization turn, functional turn(s), and encircling turn. As illustrated in Figure 2E, the pitch 254, or spacing between primary cuts 202, and depth 256 of primary cuts 202 varies along the length. By varying pitch 254, various embodiments alter the diameter of sections of docking device 250. For example, a larger pitch for primary cuts 202 allows for a larger diameter in the atrial turn, while a smaller pitch allows for a smaller diameter in the functional turn(s). Additionally, by varying depth 256 of primary cuts 202, various embodiments alter the flexibility of sections. As illustrated in the encircling turn section, the increased depth 256 of cuts 202 allow for increased flexibility for an active tip that can maneuver through native valvular anatomy (e.g., leaflets, chordae, etc.).

In various embodiments, pitch 254 varies between 1.5mm and 2.5mm (±0.25mm) to form various diameters in the various segments of the docking device 250. In some embodiments, the encircling turn(s) possess a pitch 254 of approximately 1.5mm to 2.0mm (±0.25mm) to possess a smaller diameter to surround the leaflets, while a stabilization turn possess a pitch of approximately 2.25mm to 2.5mm (±0.25mm) to have a wider diameter to maintain stability on the atrial plane. In some embodiments, the atrial turn possesses a pitch of 2.33mm. Additionally, various embodiments have an encircling turn with subsections with different pitches 254, such that allow for better fit around anatomy and/or to prevent damage to the native anatomy. In some of these embodiments, a proximal subsection possesses a smaller pitch as compared to a distal subsection. In some embodiments, the proximal subsection possesses a pitch of approximately 1.5mm (±0.25mm), while the distal subsection possesses a pitch of approximately 1.92mm (±0.25mm). Additionally, several embodiments include transition zones within a docking device 250. Such transition zones are segments where pitch 254 and/or depth 256 of cuts 202 vary to allow a smooth transition in diameter between other segments (e.g., functional turn(s) and atrial turn or functional turn(s) and encircling turn).

The length of each segment (e.g., from a segments proximal end to its distal end) can be set to optimize the region for its purpose. For example, the stabilization turn can have a length of approximately 60-70mm (±5mm) to allow for the docking device 250 to be properly secured or stabilized in the atrium. In some emboidments, the stabilization turn is approximately 68.3mm (±1.0mm). Additionally, the functional turn region can vary depending on the number of turns within this section of docking device 250. For example, the functional turn region can be approximately 65mm (±5mm) for a single turn, 130mm (±10mm) for two turns, or 195mm (±15mm) for three turns. Additionally, the encircling turn section can be any suitable distance to encircle the native anatomy. In some embodiments, the proximal subsection is 12.6mm (±2.5mm), while the distal subsection is approximately 10.0mm (±2.5mm). Finally, transition zones can be of any length to provide a suitable transition between other segments. In some embodiments, the proximal transition zone is 24.9mm (±5mm), while the distal transition zone is 10.0mm (±5mm).

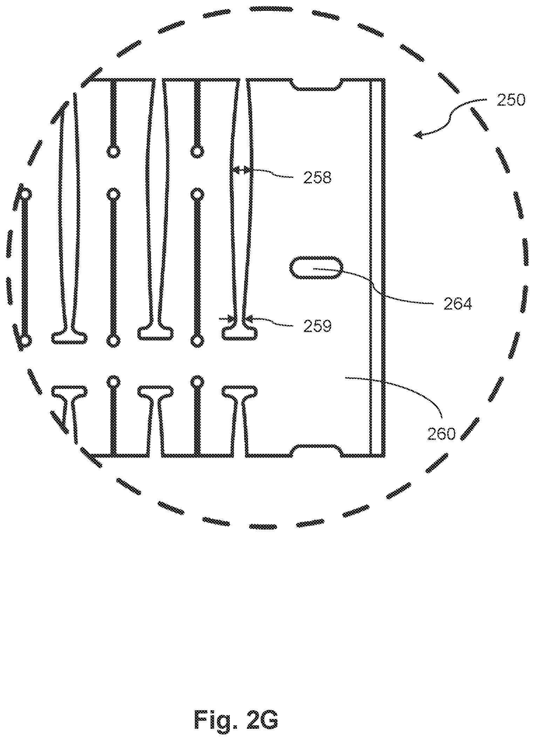

Turning to Figures 2F-2G, a flattened version of docking device 250 is illustrated, where Figure 2G illustrates an enlarged view of the circled portion of Figure 2F. Specifically, the tubular body of docking device 250 is flattened such that the tubular body is continuous across edge A and edge B. As illustrated in Figure 2F, various embodiments comprise a plurality of secondary cuts encircling the docking device 250 spaced apart by a small section of docking device 250. While Figures 2F-2G illustrate the use of two secondary cuts 203 to encircle docking device 250, additional embodiments comprise three, four, five, six, or more, secondary cuts 203 to encircle docking device 250. Secondary cuts 203 can be of any size to allow flexibility in the docking device during deployment. In certain embodiments, the secondary cuts 203 are approximately 0.06mm (±0.05mm) in width.

In addition to pitch, primary cuts 202 can vary in depth 256, and/or other dimensions (e.g., angle, width, etc.) to provide a different diameter or flexibility to a docking device 250. As seen in Figure 2F, the backside of shallower primary cuts 202' are separated by a larger section of docking device 250 than deeper primary cuts 202". Deeper cuts can provide increased flexibility of some docking devices 250. In some embodiments, the section of docking device 250 that separates apices of primary cuts is a better measure of depth for construction. In some embodiments, the section separating primary cut apices ranges from approximately 0.8mm to 2.4mm (±0.25mm), given a docking device having a tubular body with a cross-sectional outer diameter of 2.1mm. However, such distances may vary in embodiments, where a tubular body with a larger or smaller cross-sectional outer diameter. In some embodiments, the cross-sectional outer diameter of the tubular body is between 1.75 and 2.5mm (±0.25mm).

Furthermore, primary cut 202 dimensions of various embodiments vary, such as basal width or width at its narrowest point 259. In some embodiments, basal width 258 varies between 0.25 and 0.5mm (±0.15mm). In certain embodiments, basal width is 0.33mm. In many embodiments, primary cuts narrow to approximately 0.10mm (±0.05mm).

Additionally, one or both of the distal tip 260 (e.g., active tip or encircling turn) or proximal tip 262 (e.g., attached to a dock delivery system or closer to a dock delivery system) include one or more pores 264, or holes to allow attachment of additional features, such as sutures, a covering, an actuating wire, disconnecting device, or any other additional feature that can be used in conjunction with docking device 250.

### Dock Delivery System

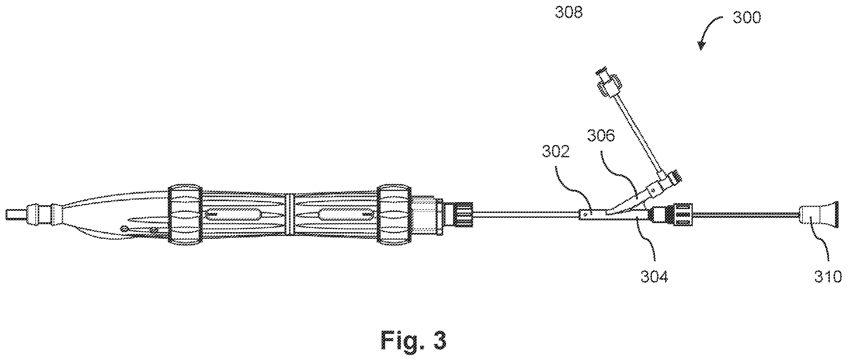

To manipulate an actuating wire of many embodiments, the actuating wire may be connected to an actuating handle on a dock delivery system, which will allow for a medical professional to manipulate the wire during dock delivery. Turning to Figure 3, a proximal end of an exemplary dock delivery system 300 is illustrated. Dock delivery systems in accordance with various embodiments are used to deliver a docking device (e.g., Figure 1, docking device 100) to its appropriate location in a heart (e.g., mitral valve, tricuspid valve). Many embodiments of dock delivery system 300 possess a delivery catheter for insertion and navigation to a heart. Additional embodiments possess a pusher shaft disposed within the catheter that is coupled to a proximal end of a docking device (e.g., Figure 1, docking device 100). In various embodiments, an actuating wire runs coaxially inside the pusher shaft to allow operation of pusher shaft and actuating wire in parallel.

Many embodiments of dock delivery system 300 possess a control assembly 302 to manipulate and/or deliver a docking device. As illustrated in Figure 3, the control assembly 302 of some embodiments comprises a hub assembly. In certain embodiments, the hub assembly is constructed as a generally Y-shaped connector comprises a straight section 304 and at least one branch 306 (though it can include more than one branch). The hub assembly or Y-shaped connector can be adapted and/or configured to allow a proximal extension of a pusher shaft to extend to the end of branch 306. Additionally, branch 306 may comprise additional components to assist in delivery of a docking device, including one or more flush ports 308. It should be noted that in embodiments that do not comprise a Y-shaped connector, a pusher shaft may connect to the strait section 304, and one or more flush ports can be located on the straight section 304. Additionally, an actuating wire extends to wire actuating handle 310 at the end of the straight section 304. With this configuration, a medical professional operates the deployment of the docking device by manipulating the position of the control assembly 302. Manipulating and/or maneuvering the dock can be accomplished by pushing and/or pulling wire actuating handle 310 - e.g., manipulation of the actuating handle manipulates a bias of the docking device. The wire actuating handle 310 and control assembly 302 can be configured to work together such that they can be moved simultaneously together when deploying and positioning the docking device at the native valve (e.g., by moving the entire hub assembly and/or Y-shaped connector forward and/or backward), but can also move independently so the pusher/pusher shaft can hold the docking device in position while docking device is maneuvered/manipulated by moving actuating handle 310 (e.g., by holding the actuating assembly 302 and/or Y-shaped connector in place relative to the main shaft of the delivery system and/or other parts of the delivery system and/or docking device while pulling proximally on the wire actuating handle 310 manipulate/maneuver a docking device). The actuating wire and pusher shaft can be coaxial along some, all, or a majority of the delivery system to facilitate this working together.

In many embodiments, pulling actuating handle 310 up to a certain point will allow for the wire to lock into a position, such that the dock will lock into a functional shape to anchor a prosthetic valve (e.g., Figure 1). In various embodiments, the locking mechanism is a ratcheting system, where a segment of teeth on an actuating wire interact with a series of teeth located within the tube of a docking device, or within he delivery system. In further embodiments, the locking mechanism is a cam hook or camming device system to securely hold an actuating wire at a position. In certain embodiments, the locking mechanism is reversible, so that a medical practitioner can unlock and continue maneuvering a docking device via an actuating wire.

In many embodiments, the delivery of a docking device is accomplished by advancing a delivery catheter to be adjacent to a native valve. While docking devices described herein are applicable to a number of valves within a heart, a specific embodiment for delivery to the mitral valve is described. In some embodiments, the delivery catheter is advanced to the right atrium and moved into the left atrium via a transseptal puncture to position the delivery catheter to the left atrium to be positioned adjacent to the native mitral valve. Although numerous methods exist to position the delivery device in the right atrium, various embodiments will navigate the delivery catheter through transfemoral delivery, such that the delivery device navigates through the femoral vein through the inferior vena cava, prior to a transseptal puncture into the left atrium.

Once positioned adjacent to the mitral valve, pushing actuating assembly (e.g., Figure 3, actuating assembly 302) forward, a pusher shaft advances a docking device (e.g., Figure 1, docking device 100) through a native valve commissure (e.g., mitral commissure) and into the ventricle. As a docking device is advancing, a pushing or pulling on an actuating handle (e.g., Figure 3, actuating handle 310) allows for the manipulation of a docking device to maneuver through the native anatomy (e.g., leaflets and chordae) via an active tip, such as described elsewhere herein, controlled by an actuating wire. The active tip will allow an encircling turn (e.g., Figure 1, encircling turn 106) to avoid ventricular walls and/or trabeculae. Upon navigating a docking device through native anatomy, tension on an actuating wire can be increased to allow for the docking device functional turn, to achieve its desired geometry, including holding a functional turn (e.g., Figure 1, functional turn 102) to a desired diameter around native valvular anatomy. In various embodiments, an actuating wire is locked to hold the tension, thus maintaining the geometry of a docking device. Once a docking device has been fully deployed, the actuating wire is disconnected or severed to allow full deployment of the docking device and removal of the delivery catheter.

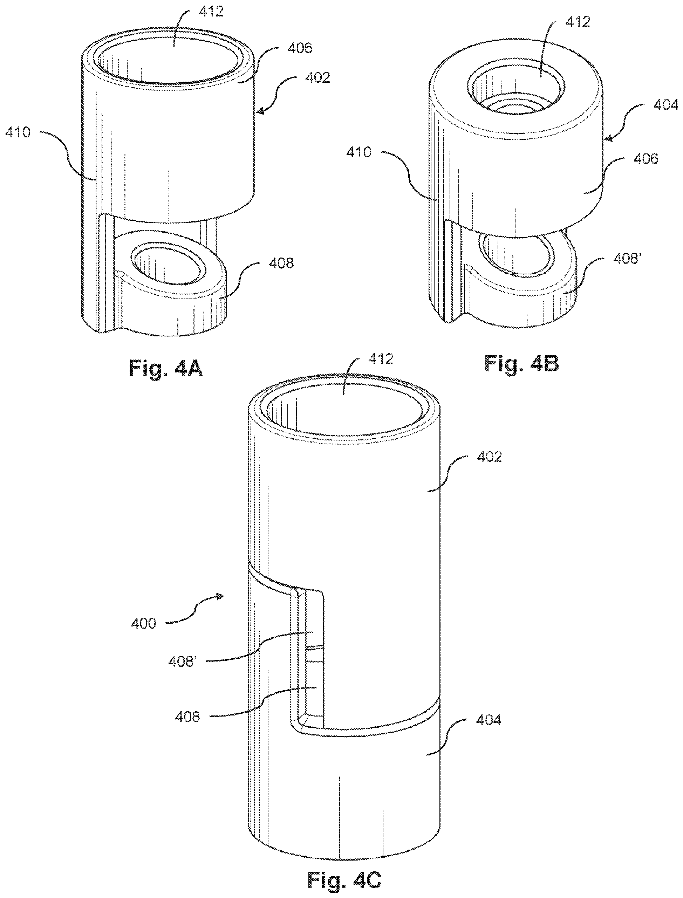

In various embodiments, an actuating wire can be disconnected from the docking device using an interlocking disconnect system. Turning to Figures 4A-4C, an interlocking disconnect device 400 of certain embodiments is illustrated. In particular, Figures 4A and 4B illustrate pusher component 402 and dock component 404 of a disconnect device 400. In various embodiments, pusher component 402 and dock component 404 each possess a connecting portion 406 and an interlocking portion 408 or 408' joined by backing portion 410. As illustrated in Figure 4C, pusher component 402 and dock component 404 interlock by interleaving interlocking portions 408, 408' from the pusher component 402 and dock component 404. Additionally, a single central pore 412 passes through both pusher component 402 and wire component 404 in the assembled interlocking disconnect device 400. By passing a wire, such as an actuating wire, through interlocking disconnect device 400, the interlocking disconnect device 400 maintains the connection between pusher component 402 and dock component 404, as a wire prevents lateral movement of pusher component 402 and dock component 404 relative to each other. Removal of an actuating wire allows for lateral movement of pusher component 402 and/or dock component 404, thus allowing the pusher component 402 and the dock component 404 of the interlocking connect device 400 to separate.

In many embodiments, pusher component 402 is affixed to a pusher shaft of a dock delivery system via connecting portion 406 by crimping, swaging, adhering, bonding, welding, or other method to secure a pusher shaft to pusher component 402. In various embodiments, the inner diameter of connecting portion 406 is sufficiently large to allow a pusher shaft with an actuating wire passing through pusher shaft to fit into the inner diameter of central pore 412.

Similarly, connecting portion 406 of dock component 404 is affixed to a docking device (e.g., Figure 1, docking device 100). In some of these embodiments, the interlocking disconnect device 400 is attached within the inner diameter of a tube forming the docking device, while certain embodiments will affix the tube of the docking device into the inner diameter of connecting portion 406. Various embodiments affix a docking device and interlocking disconnect device 400 via crimping, swaging, adhering, bonding, welding, or other method to secure interlocking disconnect device 400 to the docking device. Additionally, certain embodiments combine a locking mechanism, such as described above, into dock component 404, such that an actuating wire may freely pass through the connecting portion 406 of dock component 404, until a locking mechanism (e.g., ratcheting system, camming device, etc.) reaches its complimentary portion within connecting portion 406 of dock component 404.

Alternative embodiments may instead affix connecting portion 406 of dock component 404 to an actuating wire via a crimping, swaging, adhering, bonding, welding, or other method to secure an actuating wire into central pore 412. Additionally, as dock component 404 of these alternative embodiments does not connect to a pusher shaft, the inner diameter of connecting portion 406 needs to only be sufficiently large to connect to an actuating wire.

As described above, removal of a wire passing through interlocking disconnect device 400 allow of pusher component 402 and dock component 404 to separate from each other. In certain embodiments, the removal of a wire is effectuated by cutting or severing the wire. In certain embodiments, a cutting mechanism is installed within an interlocking connect device 400, to allow for the severing of a wire. In additional embodiments, an actuating wire can be installed with an engineered weak spot that allows for a breakage of the actuating wire by twisting or torqueing actuating wire beyond a certain threshold. In some embodiments, the twisting or torqueing is applied by a medical practitioner, such as by twisting an actuating handle (e.g., Figure 3, actuating handle 310).

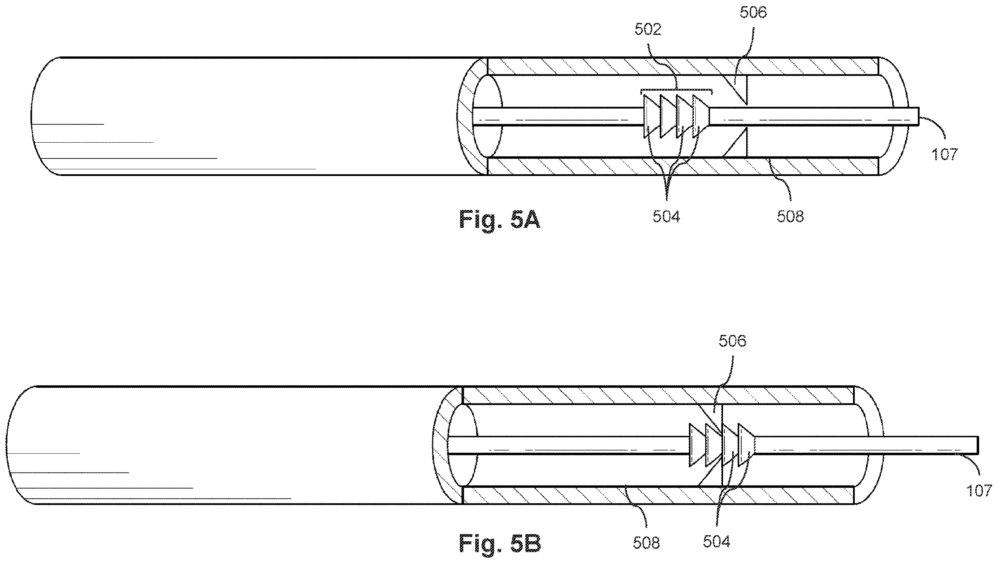

Turning to Figures 5A-5B, an exemplary locking mechanism is illustrated in accordance with many embodiments. In many embodiments, a linear rack 502 comprising one or more teeth 504 is affixed to an actuating wire 107. Additionally, many embodiments comprise one or more pawls 506 are attached to an inner surface 508 of a hollow member forming the body of a docking device (e.g., docking device 100). In embodiments, such as illustrated in Figures 5A-5B, a linear rack 502 and pawl 506 act as a ratcheting system, such that when an actuating wire 107 is manipulated beyond a certain point (e.g., when pulled or when increased tension is placed on actuating wire 107), the one or more pawls 506 engage with a tooth 504 in linear rack 502. When pawl 506 and linear rack 502 engage, actuating wire 107 is prevented from relaxing, thus locking a position of a docking device (e.g., docking device 100).

In various embodiments, the linear rack 502 circumnavigates the actuating wire 107 (e.g., surrounds 360° around actuating wire 107), while in certain embodiments, the linear rack 502 may only be attached to a portion of the wire, where the geometry or specific configuration of a docking device allows for the linear rack to be attached to 45°, 90°, 135°, 180°, 225°, 270°, or 315° around actuating wire 107. Additionally, various embodiments comprise only a single tooth 504 in linear rack 502, such that a single position is maintained of a docking device when a locking device is engaged. However, various embodiments comprise 2, 3, 4, 5, 6, 7, 8, 9, 10, or more teeth 504 in linear rack 502 to allow a practitioner to adjust the size or conformation of a docking device and/or as a security feature in case of slippage between pawl 506 and a tooth 504, such that a pawl 506 can engage with another tooth 504 rather than causing a loss of shape of a docking device or tension maintained on actuating wire 107.

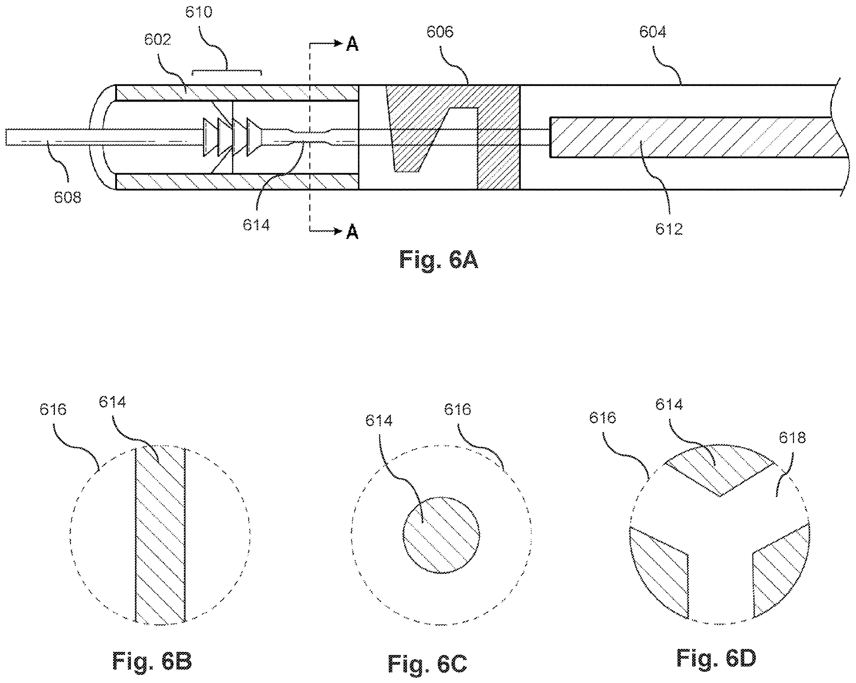

Turning to Figures 6A-6D, Various embodiments include systems or mechanisms to sever a docking device from a delivery system. Figure 6A illustrates a schematic representation of an interface of a docking device 602 with a pusher shaft 604 of a dock delivery system. In various embodiments, the docking device 602 and pusher shaft 604 interface with an interlocking disconnect device 606, such as described elsewhere herein. Additionally, as noted above, many embodiments include an actuating wire 608 that runs coaxially through docking device 602, disconnect device 606, and pusher shaft 604 and can include a locking mechanism, such as a ratchet system 610 to lock a docking device 602 into a specific conformation and/or location within the native valvular anatomy. In numerous embodiments, actuating wire 608 is attached to a torsion shaft 612 that runs coaxially within pusher shaft 604. In various embodiments, the torsion shaft 612 allows for transmission of a twisting motion from a user (e.g., physician, surgeon, other medical professional) to actuating wire 608. By transmitting torque or a twisting motion, many embodiments sever actuating wire 608. In various embodiments, actuating wire 608 comprises a detachment zone 614, at which actuating wire 608 is severed upon torsional or twisting stress. In certain embodiments, detachment zone 614 can be severed with linear or longitudinal stress along the axis formed by the actuating wire itself.

An engineered weakness within detachment zone 614 can comprise various crosssectional geometries, as identified by axis A-A. Figures 6B-6D illustrate various exemplary geometries for detachment zone 614. For example, Figure 6B illustrates a detachment zone 614 comprising a flattened cross-section versus a full diameter actuating wire, as noted by dashed line 616. Additionally, Figure 6C illustrates a detachment zone 614 with a reduced diameter versus a full diameter actuating wire 616, while Figure 6D illustrates a tripartite detachment zone 614 comprising channels 618 within a full diameter actuating wire 616. It should be noted that Figures 6B-6D illustrate examples of possible geometries for a detachment zone 614. Various embodiments incorporate additional geometries to allow for severing of an actuating wire under various tensions, forces, or stresses. In various embodiments, the detachment 614 can be created by various means of manufacture, such as by removing portions of actuating wire or by generating specific sections of wire with such geometries natively, such as through tighter weave, thinner filament within a portion, and/or by adjusting a twist or braid during manufacture of actuating wire.

### General Considerations

For purposes of this description, certain aspects, advantages, and novel features of the embodiments of this disclosure are described herein. The disclosed methods, apparatus, and systems should not be construed as being limiting in any way. Instead, the present disclosure is directed toward all novel and nonobvious features and aspects of the various disclosed embodiments, alone and in various combinations and sub-combinations with one another. The methods, apparatus, and systems are not limited to any specific aspect or feature or combination thereof, nor do the disclosed embodiments require that any one or more specific advantages be present or problems be solved.

Although the operations of some of the disclosed embodiments are described in a particular, sequential order for convenient presentation, it should be understood that this manner of description encompasses rearrangement, unless a particular ordering is required by specific language set forth below. For example, operations described sequentially may in some cases be rearranged or performed concurrently. Moreover, for the sake of simplicity, the attached figures may not show the various ways in which the disclosed methods can be used in conjunction with other methods. Additionally, the description sometimes uses terms like "provide" or "achieve" to describe the disclosed methods. These terms are high-level abstractions of the actual operations that are performed. The actual operations that correspond to these terms may vary depending on the particular implementation and are readily discernible by one of ordinary skill in the art.

As used in this application and in the claims, the singular forms "a," "an," and "the" include the plural forms unless the context clearly dictates otherwise. Additionally, the term "includes" means "comprises." Further, the terms "coupled" and "associated" generally mean electrically, electromagnetically, and/or physically (e.g., mechanically or chemically) coupled or linked and does not exclude the presence of intermediate elements between the coupled or associated items absent specific contrary language.

As used herein, the term "proximal" refers to a position, direction, or portion of a device that is closer to the user and further away from the implantation site. As used herein, the term "distal" refers to a position, direction, or portion of a device that is further away from the user and closer to the implantation site. Thus, for example, proximal motion of a device is motion of the device toward the user, while distal motion of the device is motion of the device away from the user. The terms "longitudinal" and "axial" refer to an axis extending in the proximal and distal directions, unless otherwise expressly defined.

In view of the many possible embodiments to which the principles of the disclosed technology may be applied, it should be recognized that the illustrated embodiments are only preferred examples and should not be taken as limiting the scope of the disclosure. Rather, the scope of the disclosure is at least as broad as the following claims.

The application further comprises the following embodiments:
1. A docking device for docking a prosthetic valve at a native heart valve, the docking device comprising: a coil constructed of a hypotube and extending along a central axis, including an encircling turn, a central region, and a stabilization turn, wherein: the central region possesses approximately one turn, the encircling turn extends from one end of the central region and has a diameter around the central axis greater than a diameter of the central region, and the stabilization turn has a diameter around the central axis greater than the diameter of the central region and extends from an opposing end of the central region from the leading turn.
2. The docking device of embodiment 1, wherein the hypotube comprises a plurality of cuts to allow biasing of the hypotube.
3. The docking device of embodiment 2, wherein at least one of a width and a depth of cuts on one side of the hypotube allow for biasing of the hypotube.
4. The docking device of embodiment 2, wherein at least one of a width and a depth of cuts on one side of the hypotube are varied along the length of the hypotube allowing for varied biasing of the hypotube along its length.
5. The docking device of embodiment 2, wherein a pitch is varied between cuts to allow for biasing of the hypotube.
6. The docking device of embodiment 5, wherein the pitch varies between 1.5mm and 2.33mm.
7. The docking device of any of embodiments 5-6, wherein the stabilization turn has a pitch of approximately 2.33mm.
8. The docking device of any of embodiments 5-7, wherein the central region has a pitch of approximately 1.5mm.
9. The docking device of any of embodiments 5-7, wherein the central region has a pitch of approximately 2.0mm.
10. The docking device of any of embodiments 5-9, wherein the encircling turn has a pitch of between approximately 1.5mm and approximately 1.92mm.
11. The docking device of embodiment 2, wherein the encircling turn comprises a proximal subsection and a distal subsection, wherein the proximal subsection has a pitch of approximately 1.5mm, and the distal subsection has a pitch of approximately 1.92mm.
12. The docking device of embodiment 2, wherein the coil further includes a transition zone between the central region and the stabilization turn.
13. The docking device of embodiment 12, wherein at least one of a pitch and a depth of cuts are varied along the length of the transition zone.
14. The docking device of embodiment 2, wherein the coil further includes a transition zone between the central region and the encircling turn.
15. The docking device of embodiment 14, wherein at least one of a pitch and a depth of cuts are varied along the length of the transition zone.
16. The docking device of any of embodiments 1-15, further comprising an atraumatic covering disposed on the coil.
17. The docking device of embodiment 16, wherein the atraumatic covering is constructed of PET or ePTFE.
18. The docking device of any of embodiments 1-17, wherein the coil is constructed of nitinol.
19. The docking device of any of embodiments 1-18, further comprising an actuating wire connected to a distal tip of the leading turn and running inside the hypotube.
20. The docking device of embodiment 19, further comprising an interlocking disconnect device connected to a proximal end of the docking device.
21. The docking device of embodiment 19 further comprising: a pawl disposed on an internal surface of the hypotube; and a tooth disposed on the actuating wire; wherein movement of the actuating wire beyond a certain point causes the pawl and tooth to engage.
22. A system to deliver a docking device at a native valve, comprising: a delivery catheter; a docking device of any of embodiments 1-9, the docking device having an end portion at the end of the stabilization turn located opposite the central region; a pusher shaft disposed in the delivery catheter and coupled to the end portion of the docking device; and an actuating wire connected to a distal end of the docking device and running coaxially with the pusher shaft; wherein the system is configured such that the pusher shaft and the actuating wire are configured to operate in parallel.
23. The system of embodiment 22, further comprising an actuating assembly, wherein the pusher shaft and the actuating wire are controlled via a control assembly.
24. The system of embodiment 23, wherein the actuating assembly comprises a generally Y-shaped connector.
25. The system of embodiment 24, wherein the generally Y-shaped connector comprises a straight section and a branch, wherein the actuating wire extends to the end of the straight section.
26. The system of any of embodiments 22-25, further comprising an actuating handle connected to the actuating wire, wherein manipulation of the actuating handle manipulates a bias of the docking device.
27. The system of any of embodiments 22-26, further comprising an interlocking disconnect device disposed between the pusher shaft and the docking device.
28. The system of embodiment 27, wherein the interlocking disconnect device comprises a pusher component and a dock component, each comprising a connecting portion and an interlocking portion, wherein the interlocking portion of the pusher component and the interlocking portion of the wire component interleave.
29. The system of either embodiment 27 or 28, wherein the interlocking disconnect device forms a central pore, wherein the actuating wire passes through the central pore.
30. The system of any of embodiments 27-29, further comprising a locking mechanism to lock the actuating wire in place.
31. The system of any of embodiments 28-30, wherein the dock component is affixed to the docking device and the pusher component is affixed to the pusher shaft.
32. The system of any of embodiments 22-31, further comprising a linear rack including a tooth affixed to the actuating wire and a pawl affixed to an inner surface of the docking device, wherein the linear rack and pawl are configured to interact when the actuating wire is manipulated beyond a certain point.
33. A method to deliver a docking device, comprising: advancing a delivery catheter to a native valve; and advancing a docking device disposed within the delivery catheter through a valve commissure and around valvular leaflets.
34. The method of embodiment 33, further comprising actuating an actuating wire disposed within the docking device to maneuver the docking device around the valvular leaflets.
35. The method of any of embodiments 33-34, further comprising actuating an actuating wire to achieve a desired geometry of the docking device.
36. The method of any of embodiments 33-35, further comprising actuating an actuating wire to a point that locks the actuating wire in place.
37. The method of any of embodiments 34-36, further comprising severing the actuating wire.

## Claims

1. A docking device (100; 250) for docking a prosthetic valve at a native heart valve, the docking device (100; 250) comprising:
a coil constructed of a hypotube (200) and extending along a central axis, including an encircling turn (106), a central region (102), and a stabilization turn (104), wherein:
the central region (102) possesses at least one turn, the encircling turn (106) extends from one end of the central region (102) and has a diameter around the central axis greater than a diameter of the central region (102), and
the stabilization turn (104) has a diameter around the central axis greater than the diameter of the central region (102) and extends from an opposing end of the central region (102) from the encircling turn (107);
an actuating wire (107) connected to a distal tip of the encircling turn (106) and running inside the hypotube (200), wherein a linear rack (502) comprising one or more teeth (504) is affixed to the actuating wire (107).

2. The docking device (100; 250) of claim 1, comprising one or more pawls (506) attached to an inner surface (508) of the hypotube (200).

3. The docking device (100; 250) of claims 1 and 2, wherein the linear rack (502) and the pawl (506) act as a ratcheting system, such that when the actuating wire (107) is manipulated beyond a certain point, the one or more pawls (506) engage with a tooth (504) in the linear rack (502).

4. The docking device (100; 250) of claims 2 or 3, wherein the linear rack (502) is only attached to a portion of the actuating wire (107), where the geometry or specific configuration of the docking device (100; 250) allows for the linear rack (502) to be attached to 45°, 90°, 135°, 180°, 225°, 270°, or 315° around the actuating wire (107).

5. The docking device (100; 250) of claims 2 or 3, wherein linear rack (502) surrounds 360° around actuating wire (107).

6. The docking device (100; 250) of any of claims 1 to 5, wherein the docking device (100; 250) includes an atraumatic covering disposed on the coil.

7. The docking device (100; 250) of any of claims 1 to 6, wherein the docking device (100; 250) further includes an interlocking disconnect device (400) connected to a proximal end of the docking device (100; 250).

8. The docking device (100; 250) of any of claims 1 to 7, wherein the hypotube (200) includes a plurality of cuts (202, 203) to allow biasing of the hypotube (200).

9. The docking device (100; 250) of claim 8, wherein at least one of a width (206) and a depth (208) of the cuts (202) on one side of the hypotube (200) allow for biasing of the hypotube (200).

10. The docking device (100; 250) of claim 8 or 9, wherein at least one of the width (206) and the depth (208) of the cuts (202) on one side of the hypotube (200) are varied along the length of the hypotube (200) allowing for varied biasing of the hypotube (200) along its length.

11. The docking device (100; 250) of any of claims 8 to 10, wherein the hypotube (200) comprises a plurality of primary cuts (202) cut into one side of tube (200) that allow for deflection of the hypotube (200) toward primary cuts (202) and a plurality of secondary cuts (203) located on one or more additional sides to allow for flexibility of the hypotube (200).

12. The docking device (100; 250) of any of claims 1 to 10, wherein the coil includes a transition zone between the central region (102) and the stabilization turn (104).

13. The docking device (100; 250) of any of claims 1 to 11, wherein the coil further includes a transition zone between the central region (102) and the encircling turn (106).

14. The docking device (100; 250) of any of claims 1 to 12, wherein the inner diameter of the hypotube (200) allows the actuating wire (107) to be displaced from a central axis running longitudinally through the docking device (100).

15. A system (300) to deliver a docking device (100; 250) at a native valve, comprising:
a delivery catheter;
a docking device (100; 250) of any of claims 1-14, the docking device (100; 250) having an end portion at the end of the stabilization turn (104) located opposite the central region (102);
a pusher shaft (604) disposed in the delivery catheter and coupled to the end portion of the docking device (100; 250);
wherein the actuating wire (107) runs coaxially with the pusher shaft (604);
wherein the system (300) is configured such that the pusher shaft (604) and the actuating wire (107) are configured to operate in parallel.
